# EUROPEAN PATENT APPLICATION

(11) **EP 1 982 722 A1**
(43) Date of publication of application: **22.10.2008**
(21) Application number: 07425217.2
(22) Date of filing: 16.04.2007
(51) Int. Cl.: A61K 31/7088, A61K 31/711, A61P 13/12

(54) **Use of oligotide for the treatment of renal diseases**

(71) Applicant: Gentium S.p.A., 22079 Villa Guardia (Como) (IT)
(72) Inventor: Ferro, Laura Iris, 20121 Milano (IT); Iacobelli, Massimo, 20152 Milano (IT); Echart, Cinara, 20040 Usmate Velate (MI) (IT)
(74) Representative: Pistolesi, Roberto

(57) **Abstract**

The present study has demonstrated that heparanase expression and activity was increased in human microvascular endothelial cells and rat kidney epithelial cells growing under hyperglycemia condition, as found in diabetic nephropathy disease. Oligotide was able to:
- downregulate the heparanase gene expression;
- downregulate the cell surface protein expression;
- decrease the heparanase enzymatic activity.

Since heparanase is a critical factor in maintaining glomerular basement membrane integrity and is elevated in renal diseases, as for instance diabetic nephropathies, Oligotide should be considered for the management of these diseases.

SEQUENCE LISTING

<110>  Gentium Spa

<120>  Use of Oligotide for the treatment of renal diseases

<130>  07ap34e

<140>  07425217.2
<141>  2007-04-07

<160>  4

<170>  PatentIn version 3.3

<210>  1
<211>  19
<212>  DNA
<213>  Artificial

<220>
<223>  PCR primer

<400>  1
tcaccattga cgccaacct                                                    19


<210>  2
<211>  21
<212>  DNA
<213>  Artificial

<220>
<223>  PCR primer

<400>  2
ctttgcagaa cccaggagga t                                                 21


<210>  3
<211>  21
<212>  DNA
<213>  Artificial

<220>
<223>  PCR primer

<400>  3
tttgcagctg gctttatgtg a                                                 21


<210>  4
<211>  20
<212>  DNA
<213>  Artificial

<220>
<223>  PCR primer

<400>  4
caagagtgaa aggcccagac                                                   20

SEQUENCE LISTING

<110>  Gentium Spa

<120>  Use of Oligotide for the treatment of renal diseases

<130>  07ap34e

<160>  4

<170>  PatentIn version 3.3

<210>  1
<211>  19
<212>  DNA
<213>  Artificial

<220>
<223>  PCR primer

<400>  1
tcaccattga cgccaacct                                                    19


<210>  2
<211>  21
<212>  DNA
<213>  Artificial

<220>
<223>  PCR primer

<400>  2
ctttgcagaa cccaggagga t                                                 21


<210>  3
<211>  21
<212>  DNA
<213>  Artificial

<220>
<223>  PCR primer

<400>  3
tttgcagctg gctttatgtg a                                                 21


<210>  4
<211>  20
<212>  DNA
<213>  Artificial

<220>
<223>  PCR primer

<400>  4
caagagtgaa aggcccagac                                                   20

## Description

The scope of this study was to verify the effect of Oligotide on the activity and expression of Heparanase enzyme on human microvascular endothelial cells (HMEC) and rat kidney cell (NRK52E) in hyperglycemia condition.

### 1. Background

The kidney contains a large number of functional units, the so-called nephrons. (each nephron is composed of a glomerulus, proximal tubule, loop of henle, distal tubule). A layer of parietal epithelial cells on the basement membrane encapsulates the glomerulus, which is a specialized tuft of capillaries with the afferent and efferent arteriole at either end.

The glomerular basement membrane (GBM) is a specialized extracellular matrix produced as a thin sheet-like structure by glomerular epithelial cells. The GBM functions as a primary barrier to allow molecules to selectively cross over into the urinary space. The main components in the GBM are collagen type IV, laminin, and heparan sulphate (HS) proteoglycans (4).

Loss of negatively charged HS molecules results in an altered charge-dependent permeability of GBM. The importance of HS in the charge-dependent permeability of the GBM has been demonstrated in several studies correlating with the degree of proteinuria (13,14). It has been showed that proteinuria is associated with loss of glomerular HS in diabetic and nondiabetic renal disease.

Heparanase is an β-D-endoglycosidase, which degrades heparan sulphate (HS) side chains of heparan sulphate proteoglycans in the extracellular matrix (12; 15; 16). Heparanase plays a key role in the aberrant remodelling of the GBM (5) and has been shown to be up-regulated by hyperglycaemic conditions such as found in diabetic patients (6; 7).

Diabetic nephropathy is a major cause of end stage renal disease. It is characterized by glomerular hemodynamic abnormalities that results in glomerular hyperfiltration, leading to glomerular damage as evidenced by microalbuminuria.

As glomerular function continues to decline, over proteinuria, decreased glomerular filtration rate, and end-stage renal failure results (7; 11).

A recent study in patients with diabetic nephropathy suggested that loss of HS in the GBM is attributable to accelerated HS degradation by increased heparanase expression (11). Studies in experimental renal diseases, such as passive Heyman nephritis, puromycin aminonucleoside nephrosis, and anti-GBM nephritis, suggest that heparanase also may be involved in nondiabetic proteinuric diseases. (7; 8; 9; 10).

In the present study we address the question whether Oligotide is effective to regulate the expression and activity of heparanase in human microvascular endothelial cell (HMEC) and rat kidney epithelial cell lines (NRK52E) growing in hyperglycemia conditions. The Heparanase activity and its possible inhibition can be determined by the heparan Degrading Enzyme Assay Kit whereas, its expression and possible down regulation can be valuated by Real-Time PCR and cytometric analysis.

### 2. Definitions

The term Oligotide is herein used to identify any oligodeoxyribonucleotide having a molecular weight of 4000-10000 Dalton. Preferably it identifies any oligodeoxyribonucleotide or mixture of oligodeoxyribonucleotide oligodeoxyribonucleotides having the following analytical parameters:
- molecular weight (mw): 4000-10000 Dalton;
- hyperchromicity (h): <10;
- A+T/C+G: from 1.100 to 1.455;
- A+G/C+T: from 0.800 to 1.160;
- specific rotation: from +30° to +46.8°, preferably from +30° to +46.2°.

The oligotide may be produced by extraction from animal and/or vegetable tissues, in particular, from mammalian organs, or may be produced synthetically.

Preferably, when produced by extraction, it will be obtained in accordance with the method described in (1), (2), and (3) which are incorporated herein by reference. The oligotide is known to be endowed with a significant anti-ischemic activity.

### 3. Description of method

To evaluate the effect of Oligotide either on Heparanase expression and its activity, the HMEC and NRK52E cells were incubated for 24h with DF at different concegrowing under standard (glucose 5mM) and hyperglycemia conditions (glucose 30mM) for 5 days with and without Oligotide at concentration of 150µg/ml. Then, those cells were washed with phosphate-buffered saline (PBS) pH7.4, and HMEC and NRK52E samples were prepared for different experiments.

### 3.1. Cell culture

The HMEC was kindly provide by the Regensburg University (Munich, Germany) and propagated in RPMI medium, supplemented with 10% fetal calf serum (FCS). The NRK52E cell line was purchased from American Type Culture Collection and propagated in DMEM medium, supplemented with 10% FCS.

### 3.2. Real Time PCR

### 3.1.1. RNA isolation:

RNA has been isolated from HMEC (1.5x10⁵ cells/ml) and NRK52 (1x10⁵ cells/ml) cells grew under standard (glucose 5mM) and hyperglycemia conditions (glucose 30mM) for five days with or without Oligotide at concentrations of 150µg/ml. To isolate the RNA were used the RNeasy Mini Kit from Qiagen according the manufacture's instructions.

The 1% agarose gel electrophoresis, stained with Ethidium Bromide, was performed on all samples to check for presence of clear 28S and 18S ribosomal subunit bands.

### 3.1.2. cDNA synthesis:

Purified RNA, was used as a substrate for single-stranded cDNA synthesis using iScript^{™}CDNA Synthesis Kit (Bio-Rad) including: MuLV reverse transcriptase, random examers and dNTP mix. The incubation was carried out at 42°c for 30 min. The template is the cDNA generated from reverse transcription reaction.

### 3.1.3. Real-Time PCR:

In order to perform the Real Time PCR was used the SYBER Green PCR Master Mix Reagent (SYBER Green PCR- Bio-Rad). Direct detection of polymerase chain reaction (PCR) product was monitored by measuring the increase in fluorescence caused by the binding of SYBER Green to double-stranded DNA.

Real Time PCR, using specific primers for human Heparanase [forward 5'- TCACCATTGACGCCAACCT-3' (SEQ ID NO 1); reverse 5'-CTTTGCAGAACCCAGGAGGAT-3' (SEQ ID NO 2)] and rat heparanase [forward 5'-TTTGCAGCTGGCTTTATGTG-3' (SEQ ID NO 3); reverse 5'-CAAGAGTGAAAGGCCCAGAC-3' (SEQ ID NO 4)], was performed on the MyIQ PCR Sequence Detection System (Bio-Rad) designed for used with the SYBER Green PCR master mix reagents. The cycling parameters was 95°C for 3 min, 45 cycles at 95°C; 45°C; 72°C for 30s each and 72°C for 5 min. Data were acquired and processed with the MyIQ PCR software. The housekeeping actin transcript was used to normalized for the amount and quality of the RNAs.

### 3.3. Flow Cytometry analysis:

Cell surface expression of heparanase in HMEC and NRK52E cells growing under standard (glucose 5mM) and hyperglycemia conditions (glucose 30mM) for five days with or without Oligotide (150µg/ml) were evaluated by immunofluorescence using flow cytometry.
Cells (1x10⁶ Cell/tube) were incubated with 100µl of polyclonal antibody to heparanase or only secondary antibody as negative control on ice for 1h. After incubation with secondary antibody for 1h and washing twice with cold phosphate buffer solution (PBS), cells were analysed on Becton Dickinson FACS calibur flow cytometer.

### 3.4. Heparanase Activity assay:

The Heparanase activity was measured in HMEC and NRK52E cells extracts (1x10⁵ Cell/ml of extraction buffer) by a commercial Heparan Degrading Enzyme Kit (Takara-bio Inc.) according to manufacturer's instruction. Those cell lines were growing under standard and hyperglycemia conditions for five days with saline (control) or oligotide at doses of 150µg/ml.

### 3.3.1. Principle of method:

Heparan Degrading Enzyme Assay Kit measure the activity of heparan degrading enzyme in cultured cells, utilizing the property that heparan-like molecules and bFGF (basic fibroblast growth factor) combine each other. CBD-FGF is a fusion protein of cell-binding domain of human fibronectin and human fibroblast growth factor (Takara-bio Inc.). This CBD-FGF is bound on a microtiterplate supplied in this kit, with captured by anti-fibronectin antibody having epitope in CBD region.

In addition, biotinylated heparan sulfate is used as a substrate of the enzyme. Since only undegraded substrate can combine to CBD-FGF, the detection of the remaining undegraded substrate by avidin-peroxidase realizes high sensitive measurement.

The reaction has been performed following the schematic steps bellow:
- Reaction of biotinylated heparan sulfate and sample
- Transfer of the reactant into well of CBD-FGF immobilized 96-well plate
- Reaction of remaining undegraded biotinylated heparan sulfate bound to CBD-FGF with avidin POD conjugate
- Color development by POD substrate

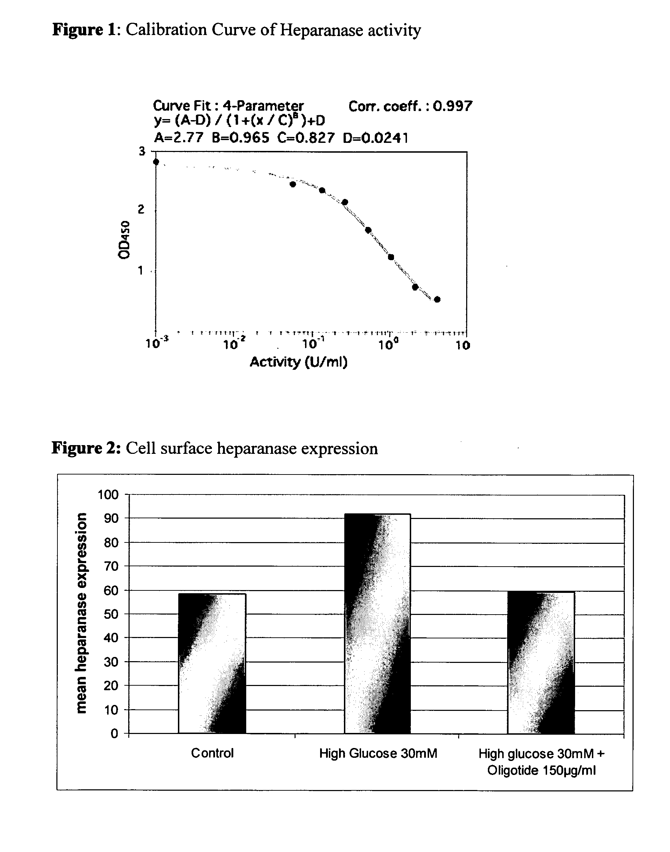

The calibration curve of Heparanase activity is reported in figure 1.

### 4. Results

### 4.1. Effect of Oligotide on Heparanase gene expression

Real-Time PCR was performed on cDNAs prepared from confluent HMEC and NRK52E cells growing under standard or hyperglycemia condition treated with saline (control) or oligotide at dose of 150µg/ml. The experiments were performed in triplicate and the results are expressed as mRNA levels normalized by the housekeeping actin gene.

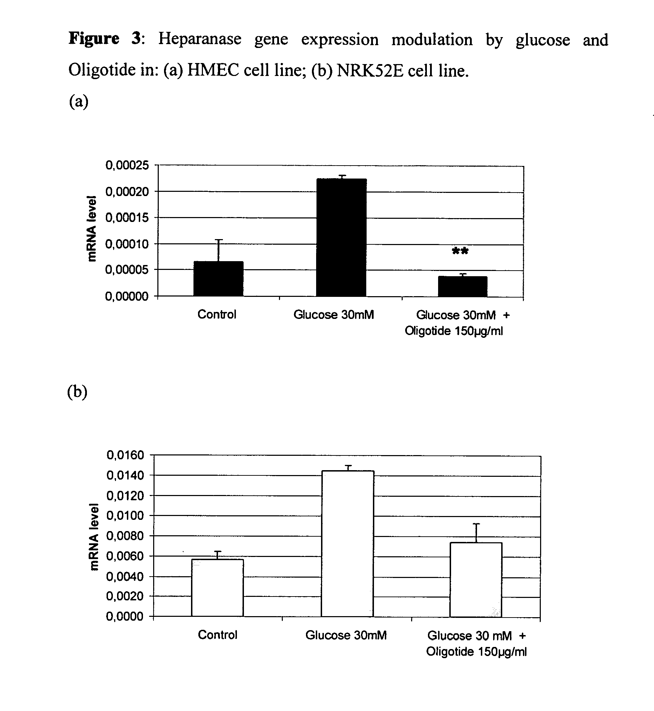

Our results, which are summarized in figure 3, showed a significant increase of heparanase gene expression in HMEC and NRK52E cell lines cultured in high glucose concentration compared with standard conditions. Oligotide treatment at dose of 150µg/ml reversed the upregulation of heparanase gene expression

### 4.2. Effect of Oligotide on Heparanase expression - FACS analysis

Flow cytometric analysis was performed to test whether the increase of cell surface heparanase expression may cause be cause by hyperglycemia and verify the effect of Oligotide. The experiments in NRK52E cells were performed using polyclonal antibody to heparanase and only secondary antibody as negative control.

As shown in figure 2, oligotide was able to reverse the glucose effect on increase the heparanase expression on NRK52E cells.

### 4.3. Effect of Oligotide on enzymatic activity of Heparanase in HMEC and NRK52E cell lines.

The activity of Heparanase were measured using the Heparanse Degrading Enzyme Assay kit on HMEC cells treated with saline (control) or Oligotide at dose of 150µg/ml growing under hyperglycemia condition. The experiments were performed in triplicate and the activity of Heparanase is shown with decrease of absorbance.

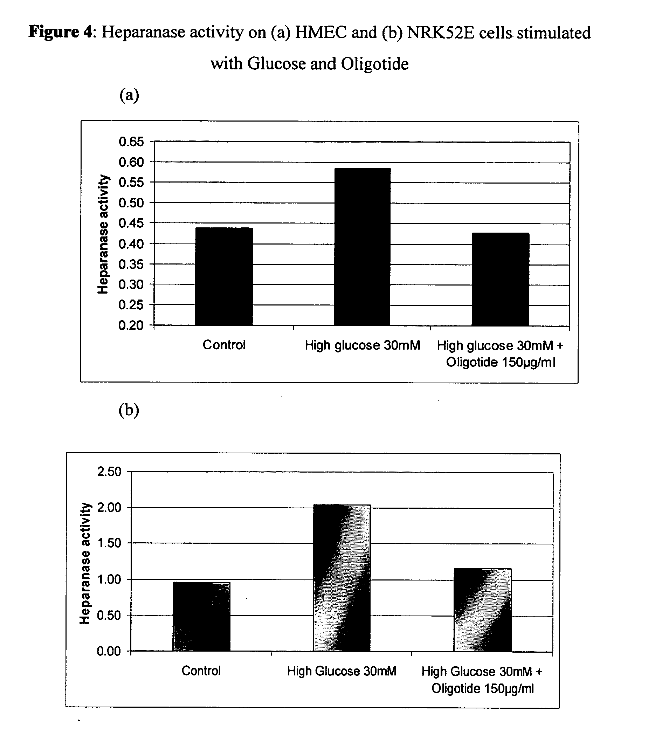

Our results, which are summarized in figure 4, demonstrate that glucose-induced an increase on heparanase enzymatic activity and the treatment with Oligotide reduce the heparanase activity in the HMEC cell line.

### 5. Conclusion

In summary, our current study demonstrated that heparanase expression and activity was increased in human microvascular endothelial cells and rat kidney epithelial cells growing under hyperglycemia condition, as found in diabetic nephropathy disease. Oligotide was able to:
- downregulate the heparanase gene expression;
- downregulate the cell surface protein expression;
- decrease the heparanase enzymatic activity.

Since heparanase is a critical factor in maintaining GBM integrity and is elevated in diabetic nephropathy, the object of the present invention is therefore represented by the use Oligotide for the for the management of this disease and, more in general, for the treatment of diseases which are positively affected by the inhibition of Heparanse and/or by the downregulation of Heparanse gene expression, such as renal diseases and, in particular, those renal diseases wherein heparanase has an over expression (such as: passive Heyman nephritis, puromycin aminonucleoside nephrosis, and anti-GBM nephritis) and/or renal diseases with proteinuria such as: systemic lupus erythematosus (i.e. SLE), minimal change disease, membranous glomerulonephritis, adriamycin nephrosis.

As regards the methods of administering Oligotide, they are not limiting for the purposes of the invention. That is to say, Oligotide can be administered to mammals (and in particular to human beings) in accordance with the methods and the posologies known in the art; generally, it may be administered orally, intramuscularly, intraperitoneally, subcutaneously or intravenously, the last-mentioned route being the preferred one.

### 6. References

1) US5646127 )
2)US5646268)
3)US6046172)
4)Conde-Knape K et.al. Diabetes Metab Res Rev; 2001 (14)412-421
5) Dempsey et al., Glycobiology, 2000; (10), n. 55, pp 467-475
6) Han et al., Cardiovascular Diabetology, 2005; (4):1-12
7) Levidiotis et al., Nephrology, 2005; (10), n. 2, pp. 167-173(7)
8) LevidiotisV et.al. J Am Soc Nephrol; 2004 (15):68-78
9) LevidiotisV et.al. J Am Soc Nephrol; 2004 (15):2882-2892
10) Levidiotis V et.al. Kidney Int, 2001; (60):1287-1296
11) Maxhimer, JB et.al. Diabetes 2005; (54):2172-2178
12) Parish, C.R. et.al., Biochem. Biophys. Acta., 2001; (1471):M99-M108
13) Tamsma JT et.al. Diabetologia; 1994 (37):313-320
14) van den Born J et.al. Kidney Int; 1993 (43):454-463
15) Vlodavsky, I. et.al., Nature Medicine. 1999; (5):793-802
16) Vlodavsky, I. and Friedman, Y., Clin. Invest. 2001; (108):341-347

## Claims

1. Use of an oligodeoxyribonucleotide or of a mixture of oligodeoxyribonucleotides having a molecular weight of 4000-10000 Da for the manufacture of a pharmaceutical formulation for the treatment of renal disease.

2. Use according to claim 1, **characterized in that** said renal disease is associated to proteinuria.

3. Use according to claim 1, **characterized in that** said renal disease is selected from diabetic nephropathy, passive Heyman nephritis, puromycin aminonucleoside nephrosis, anti-GBM nephritis, systemic lupus erythematosus-SLE, minimal change disease, membranous glomerulonephritis and/or adriamycin nephrosis.

4. Use according to claim 1, **characterized in that** said oligodeoxyribonucleotide or mixture of oligodeoxyribonucleotides has the following analytical parameters:
• hyperchromicity (h): <10;
• A+T/C+G: from 1.100 to 1.455;
• A+G/C+T: from 0.800 to 1.160;
• specific rotation: from +30° to +46.8°.

5. Use according to claim 4, **characterized in that** the specific rotation is from +30° to +46.2°.

6. Use according to claim 1, **characterized in that** said oligodeoxyribonucleotide or mixture of oligodeoxyribonucleotides is extracted from animal and/or vegetable tissues.

7. Use according to claim 6, **characterized in that** said oligodeoxyribonucleotide or mixture of oligodeoxyribonucleotides is extracted from mammalian organs.

8. Use according to claim 1, **characterized in that** said oligodeoxyribonucleotide or mixture of oligodeoxyribonucleotides is produced synthetically.

9. Use according to claim 1, **characterized in that** said such a pharmaceutical formulation is intended for administration to a mammalian.

10. Use according to claim 9, **characterized in that** said such a mammalian is a human being.

11. Use according to claim 1, **characterized in that** said such a pharmaceutical formulation is administered orally, intramuscularly, intraperitoneally, subcutaneously or intravenously.

12. Use according to claim 1, **characterized in that** said such a pharmaceutical formulation is an aqueous solution.
